# EUROPEAN PATENT APPLICATION

(11) **EP 1 156 109 A1**
(43) Date of publication of application: **21.11.2001**
(21) Application number: 00201698.8
(22) Date of filing: 11.05.2000
(51) Int. Cl.: C12N 15/12, C07K 14/47, C12Q 1/68

(54) **MYC targets**

(71) Applicant: ACADEMISCH ZIEKENHUIS BIJ DE UNIVERSITEIT VAN AMSTERDAM, 1105 AZ Amsterdam (NL)
(72) Inventor: Versteeg, Rogier, 3958 AA Amerongen (NL); Caron, Hubertus, Nicolaas, 1969 NG Heemskerk (NL)
(74) Representative: Prins, Adrianus Willem

(57) **Abstract**

The invention relates to the treatment and diagnosis of cancer and to the development of drugs for the treatment of cancer. The invention provides the insight that it is the myc oncogene family itself that provides for the recruitment and adaptation of the in essence normal physiological mechanisms and events to support the essentially neoplastic character of a cancer cell resulting in growth, invasion and spread. Herewith the invention provides a method for the treatment of cancer comprising modulating a *myc*-dependent downstream gene capable of supporting an essentially neoplastic character of said cancer.

## Description

The invention relates to the treatment and diagnosis of cancer and to the development of drugs for the treatment of cancer.

The normal healthy organism maintains a carefully regulated balance that responds to specific needs of the body. In particular, the balance between the creation or multiplication of new cells and the death of superfluous cells is well maintained. However, occasionally the exquisite controls that regulate cell multiplication break down and a cell begins to grow and divide although the body has no need for further cells of its type, the cell becomes essentially neoplastic whereas there is no real need for it. When the descendants of such a cell inherit the propensity to multiply without responding to regulation, the result is a clone of cells able to expand indefinitely. Ultimately, a mass called a tumour may be formed by this clone of unwanted cells; the affected individual has developed the beginning of cancer. Neoplasias or tumours arise with great frequency, especially in older individuals, but most often pose little risk to their host because they are localised. Localised tumours are generally called benign, tumours become life-threatening if they spread through the body. Such tumours are called malignant and are a further development of cancer.

The major characteristics that differentiate malignant tumours from benign ones are their invasiveness and their spread. Malignant tumours do not remain localised and encapsulated; instead they invade surrounding tissues, get into the body's circulatory system, and set up areas of proliferation away from the site of their original appearance. The spread of tumour cells and establishment of secondary areas of growth is called metastasis; the ever multiplying and spreading malignant cells have acquired the ability to metastasise.

Because apparently benign tumours may progress to malignancy and the earliest stages of malignant tumours are hard to identify, pathologists are rarely sure how a malignancy began. In any case, the cells of malignant tumours have a tendency to lose differentiated traits, to acquire an altered chromosomal composition, and to become essentially metastatic, they become invasive and spread.

A wealth of knowledge has been developed about the genetic events that transform a normally regulated cell into one that grows without responding to controls. These genetic events are generally not inherited through the gametes; rather they are changes in the DNA of somatic cells. The principal type of change is the alteration of pre-existing genes to oncogenes, whose products cause the inappropriate cell growth. Thus DNA alteration is at the heart of cancer induction and much focus has always been given in scientific research to elucidating the causative genetic events. For example, the members of the myc oncogene family play an important role in cancer. The frequency of genetic alterations of myc genes in human cancers (Dang and Lee, 1995) has allowed an estimation that approximately 70.000 U.S. cancer deaths per year are associated with changes in myc genes or in their expression. Three members, N-myc, c-myc and L-myc are rearranged, amplified, mutated and/or over-expressed in e.g. many cancers of lung, breast and colon, as well as in leukemia's and brain tumors.
The c*-myc* gene is expressed in a wide variety of tissues and tumors, while N-*myc* expression is largely restricted to embryonic tissues, pre-B cells and neuroendocrine tumors. N-*myc* is amplified in human neuroblastoma and small cell lung carcinoma and strongly expressed in Wilms' tumours and retinoblastoma. Neuroblastoma is a childhood tumor with a highly variable prognosis. Approximately 20% of neuroblastomas have N-*myc* amplification and these tumors follow a very aggressive course (Schwab *et al.,* 1983, Seeger *et al.,* 1985). Over-expression of transfected N-*myc* genes in neuroblastoma cell lines strongly increased proliferation rates (Bernards *et al.,* 1986, Lutz *et al.,* 1996). Transgenic mice over-expressing N*-myc* in neural crest-derived tissues showed a frequent development of neuroblastoma (Weis *et al.,* 1997). Numerous comparable observations have implicated c*-myc* and L*-myc* in the pathogenesis of many other tumor types (Cole, 1986, Marcu et al., 1992, Henrikson and Luscher, 1996).

The myc-family members are transcription factors with a basic/helix-loop-helix/leucine zipper (bHLHzip) domain. Heterodimers of myc and MAX proteins bind to the E-box motive CACGTG and activate target gene transcription (Blackwood *et al.,* 1992, Alex *et al.,* 1992; Ma *et al.,* 1993). The limited number of identified target genes thus far precluded the identification of myc downstream pathways. However, many experiments have suggested a role for *myc* genes in cell cycle control, metastasis, blocking of differentiation, apoptosis and proliferation rate (Henriksson and Luscher, 1996, Dang, 1999, Schmidt, 1999). Phenotypic analyses of mammalian cell lines and *drosophila* mutants with impaired myc function suggested a role for *myc* genes in cellular growth. Inactivation of both c*-myc* alleles in rat fibroblasts resulted in a 2- to 3-fold reduced growth rate (Mateyak *et al.,* 1997). Impaired *in vivo* expression of *drosophila* d*myc* retards cellular growth and results in adult flies half the normal size. A role for myc genes in growth regulation is furthermore in line with their effect on the cell cycle. Inactivation of c-*myc* in rat fibroblasts prolonged the G1 and G2 phases of the cell cycle, but not the S phase. High expression of c-*myc* or N-*myc* in human cells accelerated transition through the G1-phase (Steiner *et al.,* 1995; Lutz *et al.,* 1996). The same effect was found in *drosophila* cells, where reduced d*myc* activity increased the length of the G1 phase, while increased d*myc* expression enhanced transition through G1 (Johnston *et al.,* 1999).

Many studies have implicated expression of myc-family oncogenes in metastasis. In several tumor series there is a correlation between expression of myc genes and occurrence of metastases. This was observed for c-myc in e.g. breast cancer, bone tumor and colon cancer (Sierra et al., 1999, Gamberi et al., 1998, Kakisako et al., 1998). Experimental systems confirm a direct relationship between expression of myc genes and metastatic capacity. For instance human melanoma cells overexpressing c-myc were more metastatic than control melanoma cells (Schlagbauer-Wadl et al., 1999). However, the mechanism how expression of myc genes increases the metastatic potential of tumor cells is unknown.

Several direct targets of c-myc, as well as a series of indirectly induced genes, have been identified, but no obvious links between these genes have been found. Examples are prothymosin α (Eilers *et al.,* 1991), ornithine decarboxylase (Bello-Fernandez *et al.,* 1993), the embryonically expressed ECA39 gene (Benvenisty *et al.,* 1992), translation initiation factors elF-4E and eIF-2-alpha (Jones *et al.*, 1996; Rosenwald *et al.,* 1993), the CAD gene (Boyd *et al.,* 1997), the DEAD-box gene MrDb (Grandori *et al.,* 1996) and recently nucleolin (Greasley *et al.,* 2000). Effects on cyclins and other cell cycle regulators depend on cell type and conditions. Induction of cyclin D1 or tyrosine protein phosphatase cdc25A were found in some model systems only (Galaktionov *et al.,* 1996, Amati *et al.,* 1998, Philipp *et al.,* 1994; Daksis *et al.,* 1994; Solomon *et al.,* 1995). Also induction of cyclin E and A expression has been reported (Jansen-Durr *et al.,* 1993; Hanson *et al.,* 1994). The c-*myc* targets prothomyosin α and ornithine decarboxylase are also induced by N-*myc,* but it is unknown whether c*-myc* and N-*myc* share all their targets (Lutz *et al.,* 1996). Thus, it is clear that myc genetic alterations are central, and indeed, many of them have been identified and mapped.

However, much less effort has been spent on elucidating the supporting physiological events taking place in the ever multiplying and spreading cancer cell. That cells undergo genetic changes in their course to cancer is well understood. How these genetically changed cells recruit and adapt normal physiological mechanisms and events to support their essentially neoplastic character resulting in growth, invasion and spread, is much less well understood. For example, the myc proteins are transcription factors. They form dimers with the MAX protein and recognise the DNA sequence CACGTG. Very few target genes of the myc transcription factors have been identified thus far. The identified targets do not permit a clear understanding of the pathway that is activated by myc proteins, and therefore the biochemical role of these proteins in pathogenesis is matter of much speculation. Phenotypic observations on mammalian cell lines, transgenic mice and mutant *drosophilas* with aberrant expression of myc genes has suggested a role for myc genes in cell cycle control, metastasis, apoptosis, proliferation rate and cellular growth.

The invention now provides the insight that it is the myc oncogene family itself that provides for the recruitment and adaptation of the in essence normal physiological mechanisms and events to support the essentially neoplastic character of a cancerous cell resulting in growth, invasion and spread. Herewith the invention provides a method for the treatment of cancer comprising modulating a *myc*-dependent downstream gene capable of supporting an essentially neoplastic character of said cancer, such as growth, invasion or spread. In particular, the invention provides a method wherein said *myc*-dependent downstream gene comprises a nucleic acid essentially equivalent to one of a Tag sequence as shown in Table 1. Essentially equivalent herein relates to Tag sequences that identify similar or related genes or fragments thereof.

For example, the invention provides downstream genes that are activated or repressed by N-*myc* in human neuroblastoma. The analysis of the expression level of more than 66,233 transcripts identified 199 up-regulated and 85 down-regulated transcripts in N-myc expressing cells. Our results show that N-myc functions as a regulator of cell growth, facilitating neoplasia, by stimulating genes functioning in ribosome biogenesis and protein synthesis, as well as in mitochondrial electron transfer and ATP synthesis. Furthermore, many genes involved in cell architecture and cell-matrix interactions are down-regulated, facilitating invasion or spread of the neoplastic cell. A series of the N-myc regulated genes are target of c-myc regulation as well.
The invention herewith provides a list of MYC-family target genes with a large number of identified targets which permits the identification of pathways induced or inhibited by myc-family genes. However, with the identification of individual genes several other practical applications are provided as well. The findings can be used for e.g. development of new drugs, refined use of known drugs and recombinant technology. Some examples are described below.

For example, the invention provides assays for high-throughput screening of drugs specifically inhibiting myc-proteins or myc-downstream pathway proteins. It is well established that an individual cancer is caused by mutations in several oncogenes and/or tumour suppressor genes. Tumors of one and the same tissue can arise from different combinations of mutated oncogenes and tumor suppressor genes. The type and combination of gene defects determine the biology of the tumor cells, and thus the clinical behavior of the tumor. Future tumor therapies will be tailor made for the tumor of individual patients. Upon diagnosis, the type of oncogene activations in a tumor will be established and this will guide the choice of therapy. About 70.000 cancer deaths per year in the U.S. are associated with defects in myc genes or over-expression of these genes (Dang and Lee 1995, Dang, 1999). There are presently no drugs specifically blocking the action of myc proteins. Such drugs can now be identified in high-throughput systems, using target genes as provided by the invention, where thousands of compounds can be tested for a specific inhibitory effect on myc proteins. These test-systems need a very strict read-out system. An example of such a system used to identify inhibitors of the TP53 tumor suppressor protein was recently published (Komarov et al., 1999). A lacZ reporter construct was brought under the control of a promoter of a gene known to be induced by TP53. A mouse cell line harboring this construct was used to test 10.000 synthetic chemical compounds. The compounds were added to tissue culture wells with these cells and the few compounds inactivating TP53 were identified by a reduced expression of the LacZ reporter gene.

Our identification of the series of myc-target genes enables a sophisticated approach to identify myc-inhibitory drugs. There is presently no sensitive read-out system to identify such drugs. Some target genes of c-myc and/or N-myc have been identified, but their expression levels are only a few times modulated, which makes them essentially useless as read-out system (reviewed in Cole et al., 1999). Here we describe the identification of a series of genes that are strongly induced or suppressed i.e. at least 5-10 times by N-myc and/or c-myc. For example we showed that expression of the osteonectin or SPARC gene is down-modulated by N-myc from 280 transcripts per 10.000 transcripts in SHEP-2 to 14 transcripts in SHEP-21N. This 20-fold reduction is fully confirmed by Northern blot hybridization of mRNA from SHEP-2 and SHEP-21N (data not shown). Moreover, we confirmed that also at the protein level, SHEP-2 cells have much more Osteonectin protein than SHEP-2 (data not shown). The Osteonectin protein is known as a secreted protein (reviewed Lane and Sage, 1994). This makes testing of N-myc inhibitory drugs very feasible. Test-compounds can be added to SHEP cells in which N-myc expression can be induced, e.g. by a tetracyclin-inducible system. When N-myc expression is induced, Osteonectin protein levels will drop in these cells. If a compound is inhibitory for N-myc, this will block down-modulation of Osteonectin mRNA expression. These cells will continue secretion of Osteonectin protein in the tissue culture medium. Therefore, in a multi-well cell culture system, all wells with inactive compounds will have low Osteonectin production. The rare wells with a high Osteonectin concentration identify the compounds that inhibit N-myc functioning. Elisa or other protein-based detection systems can automatically detect the wells with high Osteonectin concentrations. Since not only Osteonectin, but many other down-regulated genes like IGFBP7, collagen type 4a1 and syndecan 2 are identified to be down-regulated by myc, they can be used in such assays as well. Similar assays can be designed the other way around, using genes induced by N-myc or c-myc as a read-out. Variants of such drug-testing systems can use the promotor elements of strongly N-myc-regulated genes and use them to control expression of easily detectable proteins like Green Fluorescent Protein. This will further simplify detection of myc-inhibitory drugs in high throughput systems.
Since we showed that many of the N-myc targets are targets of c-myc as well, our findings are used in assays for the identification of drugs against other members of the myc oncogene family. Inducible c-myc constructs will in many cell systems target the same genes as N-myc. Therefore the here described list of target genes for N-myc and c-myc enables the semi-automatic testing of hundreds of thousands candidate drugs and the selection of compounds that are active against myc proteins and/or their downstream pathways.

The invention also provides the application and further development of existing drugs for specific treatment of patients with N-myc or c-myc activated tumours, for example in a method of treatment for myc-related cancer. As explained, future cancer therapies will be designed to specifically inhibit those oncogenes that are actually activated in the tumor of a specific patient. Our finding provides the deliberate use of several currently known cytostatic or cytotoxic drugs in patients with a tumor caused by activation of a myc gene. A few examples are given. For example, the invention provides a method of treatment comprising using drugs interfering with nucleophosmin:
We identified nucleophosmin (B23) as a major target of N-myc and c-myc induction. Analysis of a large series of human tumours and cell lines shows that nucleophosmin mRNA levels also *in vivo* correlate with N-myc or c-myc expression. Nucleophosmin functions in ribosome biogenesis and nucleolar-cytoplasmic transport of pre-ribosomal particles. The protein is known to be translocated from the nucleolus to the nucleoplasm by several cytotoxic drugs, like actinomycin D, doxorubicin, mitomycin, toyocamycin, tubercidin, sangivamycin and mycophenolic acid (Yung et al., 1995, 1990, Chan et al., 1987, Chan 1988, 1992, Cohen and Glazer, 1985, Perlaky et al., 1997). Treatment of cells with these drugs inhibits processing of ribosomal RNA and protein synthesis and leads to cell death. Several of these drugs are clinically tested and/or applied for treatment of cancer patients.

Our finding that members of the myc oncogene family induce nucleophosmin mRNA and protein expression, marks this protein as an attractive target in the treatment of tumours with overexpression of a myc family member. This opens fundamental new opportunities for the above mentioned drugs and their analogons. These drugs were clinically tested or used on unselected patient series, without knowledge of their possible specific effect on myc-activated tumors. The patient series presumably consisted of patients with and without involvement of myc genes in their tumours. Actinomycin D is used in e.g. Wilms' tumor and rhabdomyosarcoma treatment. Some patients react better on therapy than other patients. A sizable part of Wilms' tumor and rhabdomyosarcoma patients have high N-myc or c-myc expression (Nisen et al., 1986). It can now be tested whether actinomycin D is specifically effective against N-myc or c-myc expressing tumors, wherein actinomycin D is ineffective against tumours that have no myc activations. These findings provide a more specific use of actinomycin D. Several other drugs are effective against nucleophosmin (see above). Improvement of these drugs by development of less toxic analogs that have more specific anti-nucleophosmin effects is now an interesting strategy provided here to design specific anti-myc drugs. E.g. toyocamycin is highly toxic (Wilson, 1968), but analogs can be tested and further developed for specific use in patients with myc-activated tumors.

The invention also provides use of inhibitors of extracellular and transmembrane proteins. The list of genes induced by N-myc and/or c-myc includes many genes coding for secreted or cell surface proteins. Such proteins are excellent targets for drugs, as they are readily accessible. They may offer targets to specifically inhibit growth or metastasis of tumour cells with an activation of myc-family members. Examples of potential targets are basigin and Plasminogen Activator Inhibitor type 1(PAI)(No. 112). Basigin (or EMMPRIN, extracellular matrix metalloproteinase inducer)(table 1, No. 97), is a member of the immunoglobulin family that is present on the surface of tumor cells and stimulates nearby fibroblasts to synthesize matrix metalloproteinases (Guo et al., 1998). Since metalloproteinases are known to promote degradation of matrix and promote metastasis, drugs that inhibit basigin would be able to prevent metastasis of tumors with high expression of myc-family genes. Drugs inhibiting metalloproteinase are known and could now be applied for specific treatment of tumors with activation of myc-family members.

Plasminogen activator inhibitor type-1 (PAI-1) is a major physiological inhibitor of fibrinolysis and matrix turnover. The here-described down-modulation of PAI-1 by N-myc would increase matrix turnover and promote cell motility ands metastasis. Many compounds are clinically and experimentally tested for regulation of PAI. For example 15-deoxy-Delta12, 14-prostaglandin J2 (15d-PGJ2), an activating ligand for the transcription factor PPARg, augmented PAI-1 mRNA and protein expression (Marx et al., 1999). It is here provided that these drugs are therefore specifically be used to prevent metastasis of N-myc or c-myc expressing tumors. Furthermore, transmembrane proteins induced by N-myc and/or c-myc proteins can be used as target of therapeutic drugs like antibodies that can be conjugated with cytotoxic drugs.

Furthermore, the invention provides further molecular diagnosis of tumors. Here is provided a first integral description of target genes of myc-family oncogenes. Several functional categories provided. However, in the analysis of fresh tumors with N-myc activation, we observed that not all these genes or all categories are up-regulated in all tumors. This suggests that additional defects or factors may affect the range of genes that are induced or repressed by myc oncogenes in individual tumors. This is likely to be of importance for the biology of the tumor. Therefore, the detailed analysis of up-regulated/suppressed genes in tumours with activation of a myc-family member is clinically relevant. A tumour with up-regulation of genes involved in protein synthesis may differ from a tumor with up-regulation of genes involved in oxidative phosphorylation. This information is important to select the appropriate treatment modality for a tumor. The full list of N-myc and/or c-myc downstream targets therefore provides an important means to classify tumors for optimal therapeutic regimens. The here-described finding can be applied to develop diagnostic kits to measure the activation or inactivation of key-downstream targets of myc-family oncogenes. Such diagnostic tools are now provided to guide the optimal therapy.

Furthermore, the invention provides non-invasive diagnosis of tumor. Activation of specific oncogenes can presently only be established by analysis of a surgically removed tumor specimen. As surgery is a burden to the patient, expensive and not without risk, non-invasive methods to monitor oncogene status of tumors would be desirable. The inventory of genes for secreted proteins that are induced or suppressed by N-myc can be used to determine the status of myc expression by analysis of serum markers. Furthermore, serum levels of these genes can be used to monitor tumor growth, reaction on therapy and occurrence of relapses. Our results shows that many candidate proteins, e.g. osteonectin (reduced from 280 to 14 tags/10.000 tags), macrophage migration inhibitory factor table 1, No. 92)(induced from 1.1 to 14.4 tags/10,000 tags) and Plasminogen activator inhibitor type 1 (PAI-1) as serum markers to aid in the biological classification of tumors. Recently serum levels of PAI-1 were analyzed in a series head and neck tumors and found to correlate with tumor stage (Strojan et al., 1998). Our findings therefore identify PAI-1 and other secreted proteins affected by myc-family members as good candidates to monitor the status of myc genes in a tumor and to follow the growth and response on therapy of myc-induced tumors.

The invention furthermore provides enhancement of cellular protein synthesis machinery for production purposes. Eukaryotic cells can be used to produce recombinant proteins, e.g. of drugs. The discovery that N-myc and c-myc induce essential components of the protein synthesis machinery can be applied to boost production of recombinant proteins in cell systems. The invention provides the use of cells with a high expression of endogenous or transfected myc genes to optimize the yields of recombinant proteins, like antibodies, hormones or other proteins with a therapeutic or commercial value.

### Detailed description

The members of the myc oncogene family play an important role in cancer. The frequency of genetic alterations of myc genes in human cancers (Dang and Lee, 1995) has allowed an estimation that approximately 70.000 U.S. cancer deaths per year are associated with changes in myc genes or in their expression. Three members, N-myc, c-myc and L-myc are rearranged, amplified, mutated and/or over-expressed in e.g. many cancers of lung, breast and colon, as well as in leukemia's and brain tumors. The myc proteins are transcription factors. They form dimers with the MAX protein and recognize the DNA sequence CACGTG. Very few target genes of the myc transcription factors have been identified thus far. The identified targets do not permit a clear understanding of the pathway that is activated by myc proteins, and therefore the biochemical role of these proteins in pathogenesis is matter of much speculation. Phenotypic observations on mammalian cell lines, transgenic mice and mutant *drosophilas* with aberrant expression of myc genes has suggested a role for myc genes in cell cycle control, metastasis, apoptosis, proliferation rate and cellular growth.

In order to identify the downstream pathways of the myc genes, we applied the SAGE (Serial Analysis of Gene Expression) technique. We identified about 66,233 tags, each representing a mRNA transcript, in a pair of N-myc transfected and control-transfected neuroblastoma cell lines. We have identified 197 tags, each representing a transcript, that are specifically induced and 85 tags that are suppressed by N-myc. N-myc appears to induce the expression of many ribosomal protein genes, genes involved in ribosomal RNA synthesis and ribosome biogenesis and genes involved in translation and protein maturation. This indicates that a major function of N-myc is the enhancement of the protein synthesis machinery of the cell. Furthermore, there is a striking induction of genes involved in glycolysis and in electron transport and ATP synthesis in the mitochondria. This suggests an increased capacity of the cellular energy production mechanism. Another set of N-myc targets is involved in cellular adhesion, matrix formation, invasive capacity and cytoskeletal architecture. These data explain the increased metastatic potential associated with myc-expressing tumor cells. Furthermore, a set of genes is induced or suppressed with a role in transcription, chromosome condensation and signal transduction. Finally, a series of genes are identified for which only a short cDNA sequence is known (Ests) and some SAGE transript tags were identified without a gene assignement. These genes may be important components of the N-myc downstream pathway. Many of these downstream targets of N-myc appear to be targets of the c-myc oncogene as well. Therefore, these data represent an inventory of the target genes of the myc oncogene family. As these genes mediate the tumorigenic effects of myc-family oncogenes, they offer the opportunity to identify new drugs that inhibit myc proteins or myc downstream pathways. Furthermore, they represent a range of potential target genes to inhibit or kill tumor cells expressing members of the myc-family of oncogenes.

### Results

SAGE libraries of N*-myc* transfected neuroblastoma cell lines

To identify the downstream target genes of N-myc, we applied the SAGE technique on an N*-myc* transfected neuroblastoma cell line. The SHEP cell line has no N-*myc* amplification and expression, nor c-*myc* expression. A tetracycline-dependent N-*myc* expression vector has been introduced in these cells, resulting in the SHEP-21N clone (Lutz *et al.,* 1996). The SHEP-21N cells have constitutive exogenous N*-myc* expression that can be switched off by tetracycline. N-*myc* expression in the SHEP-21N cells was shown to increase the rate of cell division, shorten the G1 phase of the cell cycle and to render the cells more susceptible to apoptotic triggers (Lutz *et al.,* 1996; Fulda *et al.,* 1999). Two SAGE libraries were constructed, one from SHEP-21N cells expressing N*-myc* and one from the SHEP-2 control cells. The SHEP-2 clone was transfected with the empty expression vector. From SHEP-2 we sequenced about 44,674 transcript tags and from the SHEP-21N library we sequenced 21.559 transcript tags. Comparison of the two SAGE libraries yielded 199 significantly (p<0.01) up-regulated tags in N-*myc* expressing cells, with induction levels of up to 47-fold (Table 1, section 1). Another 85 tags were significantly down regulated. Here we describe these tags and the most likely gene assignment that can presently be made. The transcripts corresponding to these tags were identified using the SAGEmap database from CGAP/NCBI (Lal *et al.,* 1999). Seven groups of N-myc regulated genes are described.

### N-myc targets 1: ribosomal protein genes

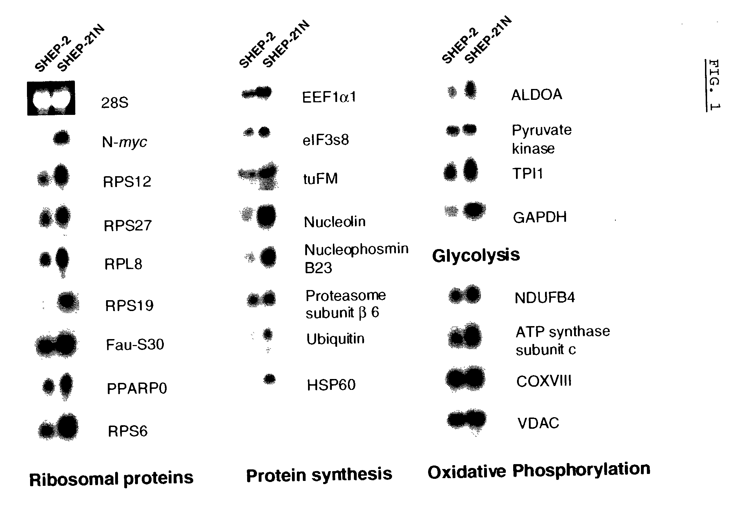

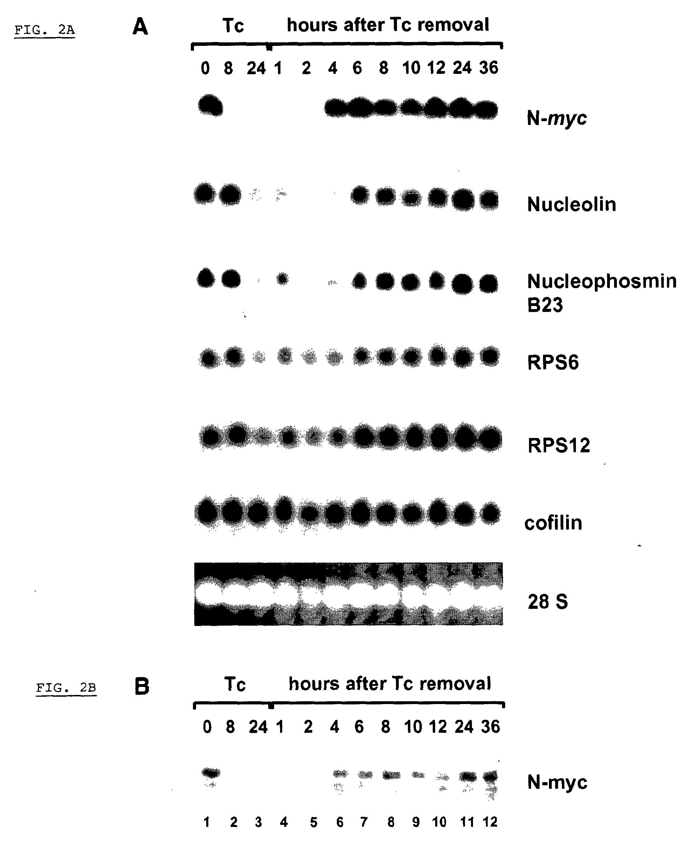

The first functional group consists of 61 ribosomal protein genes, that were induced up to 47-fold (p<0.01, Table 1, sectionl). These 61 proteins represent about 75% of the human ribosomal proteins (Wool *et al.,* 1996). Seven of the induced genes were selected for further analysis. Northern blots with equal amounts of total RNA from SHEP-2 and SHEP-21N cells were hybridized with probes for the ribosomal proteins S12, S27, Fau-S30, L8, S6, S19 and the ribosomal phosphoprotein P0 (PPARP0) (Figure 1). All seven genes were induced by N-*myc.* The total amount of tags found for ribosomal protein mRNAs comprises about 4% of all tags in SHEP-2. This fraction has increased to 10% in SHEP-21N. The level of induction of individual ribosomal protein genes is a function of their basal expression levels in SHEP-2. Highly expressed genes are less induced than genes with a low basic expression in SHEP-2 (Figure 2A). These results indicate that N-myc induces, directly or indirectly, the mRNA expression level of the majority of ribosomal proteins.

### N-myc targets 2: genes functioning in ribosome biosynthesis and protein synthesis

Also a second functional group of 26 tags corresponds to genes with a distinct role in protein synthesis and turnover, notably ribosome biogenesis, mRNA translation, protein maturation and degradation (Table III).

Highly interesting is the induction of nucleophosmin (B23) (table 1, no.'s 67 and 83). Northern blot analysis confirmed this induction (Figure 1), to a level even stronger than suggested by the tag frequencies. Nucleophosmin is a highly abundant nucleolar protein that processes ribosomal RNA by cleavage of the 5' end of the 5.8S pre-rRNA (Savkur *et al.,* 1998). It furthermore functions in assembly and nuclear-cytoplasmic shuttling of pre-ribosomal particles (Borer *et al.,* 1989; Olson *et al.,* 1991, Szebeni *et al.,* 1999). Nucleophosmin is the target of recurrent chromosomal translocations in lymphomas and leukemia (Morris *et al.,* 1994, Redner *et al.,* 1996, Pandolfi, 1996). The prominent role of nucleophosmin in ribosome biogenesis urged us to analyze the SAGE libraries for other genes implicated in this process. Nucleolin, which also has two tags due to alternative transcripts (table 1, no.'s 87 and 88), is induced from 2.5 to 5.6 tags per 10,000 in total (p=0.044). This induction was confirmed by Northern blot analysis (Figure 1). Nucleolin is also a highly abundant nucleolar protein and binds to nucleophosmin (reviewed Tujeta and Tujeta, 1998; Ginisty *et al.,* 1999). It probably is a rate-limiting enzyme for the first step in the processing of the pre-ribosomal RNA to mature 18S rRNA (Gistiny *et al.,* 1998). Nucleolin is furthermore involved in the assembly of pre-ribosomal particles and their nucleo-cytoplasmic transport. It interacts with 18 ribosomal proteins (Bouvet *et al.,* 1998), sixteen of which are induced by N-*myc*. The induction of nucleolin and nucleophosmin by N*-myc* suggests that besides ribosomal proteins, also ribosomal RNA and ribosome biosynthesis are target of N-*myc* stimulation.

Also tags corresponding to three translation initiation factors and five translation elongation factors were induced. The initiation factors are eukaryotic translation initiation factor 3 subunit 8 (eIF3s8) (No.81) and subunit 3 (No.78), eukaryotic translation initiation factor 4B (No.72). Elongation Factor 1 (EEF1), responsible for delivery of aminoacyl-tRNA to the ribosome, is a heterotrimer either consisting of the subunits alpha/beta/gamma or alpha/delta/gamma. The tags for the subunits alpha, delta and gamma are induced 9- to 11.4-fold in SHEP-21N (No.'s 69, 66 and 70). Elongation Factor 2, which promotes the translocation of the nascent polypeptide chain from the A-to the P-site of the ribosome, is also induced (No. 79). The mitochondrial elongation factor Tu (tuFM), which delivers aminoacyl-tRNA to the mitochondrial ribosomes, is 12.4 times up-regulated (No. 64). Northern blot analysis of SHEP-21N and SHEP-2 confirmed the induction of eIF3s8, EEF1a1 and tuFM (Figure 1). These data further support a role for N-*myc* as a regulator of protein synthesis.

A next step in protein synthesis is maturation and routing. The nascent polypeptide-associated complex (NAC) alpha mRNA was induced in N-*myc* expressing cells (No. 77). NAC protects nascent polypeptide chains of cytosolic proteins from inappropriate translocation to the endoplasmatic reticulum (Wiedmann *et al.,* 1994). Induction of the chaperones HSP60 and HSP90 further suggested an increased cellular capacity for protein folding and maturation (No.'s 65, 68, 80 and 82). HSP60 is implicated in mitochondrial protein import and macromolecular assembly. HSP90 is involved in the folding of a signaling molecules including steroid-hormone receptors and kinases and the refolding of misfolded proteins. Northern blot analysis confirmed the induction of HSP60 (Figure 1). Also the cellular capacity for protein degradation was possibly induced. This was suggested by the increased tag frequencies for three ubiquitin pathway proteins (No.'s 62, 73 and 76) and five proteasome subunits (No.'s 63, 71, 74, 75 and 84). Northern blot analysis confirmed the higher expression level of proteasome subunit b type 6 in SHEP-21N cells (Figure 1).

### N-myc targets 3: glycolysis genes

A third group of N*-myc* induced genes encoded key-enzymes in the glycolytic pathway (Table 1, section 4). Tags for aldolase A fructose-biphosphate (ALDOA), triosephosphate isomerase 1 (TPI1), glyceraldehyde-3-phosphate dehydrogenase (GAPDH) and pyruvate kinase are all increased (No.'s 133, 135 and 132). Other induced mRNAs encode for the metabolic enzymes 3-phosphoglycerate dehydrogenase, involved in the synthesis of serine, and sorbitol dehydrogenase that oxidizes sorbitol to fructose. Aldehyde dehydrogenase 1 functions in ethanol metabolism. Northern blot analysis confirmed the mRNA induction of ALDOA, pyruvate kinase, TPI1 and GAPDH (Figure 1). These data implicate the glycolysis as a target of N-myc stimulation.

### N-myc targets 4: Mitochondrial electron carriers and ATP synthethase

SHEP-21N shows induction of a series of tags corresponding to genes with a role in oxidative phosphorylation in the mitochondria (Table 1 section 5). Seventeen tags are significantly induced. Interestingly, five of the induced genes are mitochondrially-encoded (see for mitochondrial tag analysis Welle *et al.,* 1999).

The oxidation of NADH and FADH2 by electron transfer to O2 is performed by three protein complexes of the respiratory chain, NADH-dehydrogenase, ubiquinol-cytochrome c reductase and cytochrome c oxidase. These large complexes establish a proton gradient across the mitochondrial inner membrane, which drives the synthesis of ATP by the F-type ATP synthase complex. N-*myc* induces a series of subunits of all four enzyme complexes.

Four NADH dehydrogenase subunits, subcomplex 4 (No. 136; NDUFB4), subcomplex 7 (No. 138) and the mitochondrially encoded subunits 4/41 and 3 (No.'s 150 and 151) are induced. The induction of NDUFB4 was confirmed by Northern blot analysis (Figure 1).

One subunit of the ubiquinol-cytochrome c reductase complex was induced (NO. 136). Furthermore, subunits II, III and VIII of cytochrome c oxidase were induced in N*-myc* expressing cells. Induction of the subunit VIII (COXVIII, No. 149) was confirmed by Northern blot analysis.

Finally, N-myc induces the transcripts of subunits 6/8 of the FO segment and of two isoforms of subunit 9 (or c) of the FO segment of the stalk of the F-type ATP synthase (No.'s 137, 148, and 152). ATPase subunits 6 and 8 are encoded on an overlapping mitochondrial transcript.

In addition, several other proteins with a role in mitochondrial function are up-regulated (table 1 section 5). The voltage dependent anion channel (VDAC, No. 143) was induced 5-fold, which was confirmed by Northern blot analysis (Figure 1). VDAC forms a mitochondrial outer membrane channel that allows diffusion of small hydrophylic molecules. It plays a major role in apoptosis, as it can transfer cytochrome c to the cytoplasm, which results in caspase 9 activation.

In addition, glutathion peroxidase 4 and glutathion S-transferase p are strongly induced (No.'s 139 and 144). Glutathion readily accepts electrons and may serve as scavenger for hydrogen peroxide and organic peroxides, the inevitable artifacts produced by the electron transport chain of the mitochondria. This reaction is catalyzed by gluthatione peroxidase.

### N-myc targets 5: genes with a role in cell motility and metastasis:

A large group of tags that are either induced or suppressed by N-myc belong to genes with a role in cell motility and cell-matrix interactions (table 1, section 3). These genes encode for cytoskeletal proteins, cell surface proteins, adhesion molecules and extracellularly secreted proteins with a role in cellular matrix architecture and turn-over. Ten tags for genes in this category were significantly induced. Another 29 tags in this category are significantly down-modulated. Examples of down-regulated genes are Collagen types Ia1 (No.'s 100, 108 and 109), type IVa1 (No. 115) and type XVIIIa1 (No. 116), fibrillin (No. 121), syndecan 2 (No. 126), fibronectin (No. 122) and Osteonectin (SPARC)(No.'s 118, 123 and 125). The latter is an interesting gene, that is down-modulated from 280 to 14 tags per 10.000 tags. We confirmed down-modulation of Ostenectin, syndecan 2 and collagen IVa1 and Plasminogen activator inhibitor type 1 (No. 112) by Northern blot analysis (data not shown). The down-modulation of these genes suggests that N-myc can reduce the adherence of cells to the cellular matrix and therefore induce the motility of the cells. This is line with an enhanced metastatic potential of myc expressing tumor cells.

### N-myc targets 6: other genes.

Another group of genes that is affected by N-myc is formed by signal transduction proteins, transcription factors, chromatin factors, cyclins and other regulatory proteins. This group counts 66 significantly induced transcripts (Table 1 section 6). Examples are NM23A, NM23B, HMG I-Y, zinc finger protein 6 and (No.'s 171, 214, 162, 218). Induction of HMG I-Y, NM23A and NM23B was confirmed by Northern blot analysis (data not shown). Another group of genes for regulatory proteins or enzymes were downmodulated by N-myc (table 1, section 6). Examples are Insulin-like growth factor binding protein 7 (IGFBP7)(No. 252) and zinc-finger protein 216 (No. 248). Northern blot analysis confirmed downregulation of IGFBP7 in SHEP-2 cells compared to SHEP-21N cells (data not shown).

### N-myc targets 7: Anonymous genes (Ests)

A series of anonymous genes for which only a partial cDNA sequence is known (expressed sequence tags or Ests) are induced or down-modulated by N-myc (Table 1 section 7). The function of these genes is as yet unknown, but the finding that they are targets of myc regulation mark them as potentially important genes with a role in cancer.

### Tags of unidentified targets of N-myc.

For several tags that were differentially represented in the SHEP-2 and SHEP-21N libraries, we have as yet not identified the corresponding genes (table 1, section 8). They belong both to genes that are induced or suppressed by N-myc.

### N-myc activates downstream targets within 4 hours

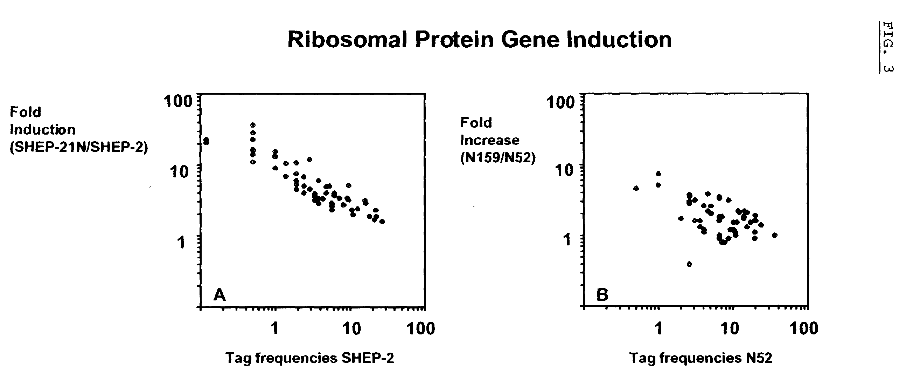

In a time-course experiment we analyzed whether the putative N-myc targets are induced after N-myc modulation in the SHEP-21N system. N-myc expression can be reversibly switched-off in SHEP-21N cells by tetracycline. SHEP-21N cells were treated for 24 hours with tetracycline, washed extensively and grown for an additional 2 to 36 hours without tetracycline. Northern blot analysis showed that the expression of N-*myc* mRNA is switched off within 8 hours of tetracycline treatment (Figure 3A, lanes 1-2). After removal of tetracyclin, N-*myc* mRNA expression is restored between 2 and 4 hours (Figure 3A, lanes 5-6). The N-myc protein expression was analyzed by Western blotting in a parallel time-course experiment and closely followed the N-*myc* mRNA expression (Figure 3B). The Northern blot filters were hybridized with probes for the N-myc downstream targets nucleolin, nucleophosmin and the ribosomal protein genes RPS6 and RPS12 (Figure 3A). After repression of N-*myc* by tetracyclin, the mRNA levels of these genes remain unaffected at 0 and 8 hrs, but their expression is reduced to low basic levels at 24 hours. Importantly, between 2 and 4 hours after re-expression of N-*myc* mRNA and protein, expression of all four genes is strongly re-induced (Figure 3B, lanes 6-7). Similar results were obtained for EEF1A1, TPI1, eIF3s8, and VDAC (data not shown). The expression level of cofilin that we used as a control does not change significantly during the time course. To exclude a direct effect of tetracyclin on nucleolin or nucleophosmin expression, we did the same experiment with SHEP-2 cells but no effect on gene expression was observed (data not shown). These results firstly confirm that the here identified genes indeed are induced by N-myc. Secondly, it shows that they are early targets in the N-myc downstream pathway, although not necessarily direct targets of N-myc. They therefore represent essential components of the N-myc pathway. The data furthermore show that the induction by N-myc is highly versatile: expression drops after N-myc abrogation and is swiftly restored after N-myc re-expression.

### N-myc induces ribosomal RNA synthesis

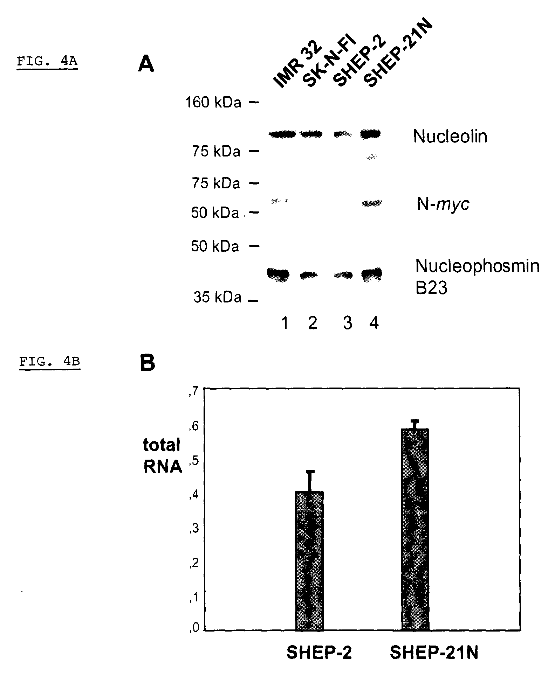

The induction of two genes with a key role in rRNA processing and ribosome biogenesis urged us to analyze their protein expression level and their possible functional activity. Protein expression of nucleolin and nucleophosmin was analyzed in SHEP-2 and SHEP-21N cells, as well as in two control cell lines with and without N-*myc* amplification. Western blot analyses showed a higher nucleolin and nucleophosmin expression in SHEP-21N compared to SHEP-2 (figure 4, lanes 3 and 4) and in the N-*myc* amplified IMR32 cell line compared to the N-*myc* single copy cell line SK-N-FI (Figure 4, lanes 1 and 2). As these proteins function in ribosomal RNA processing, we analyzed whether SHEP-21N has a higher rRNA content than SHEP-2 cells. We isolated total RNA from 10 samples of 10⁶ exponentially growing cells of each of the cell lines. Spectrophotometric analysis revealed that SHEP-21N cells have an at average 45% higher yield of total RNA than SHEP-2 cells (p<0.001, Student T test for independent samples) (Figure 4B). Duplicate experiments on independently cultured cells gave the same results. Densitometric quantification of the 18S and 24S rRNA bands fractionated by agarose gel electrophoresis confirmed that this increase is caused by ribosomal RNA (data not shown).

To analyze whether this strong increase in rRNA resulted in increased ribosomal function and overall protein synthesis, we measured protein content and the rate of protein synthesis in SHEP-2 and SHEP-21N cells. Lysates of 10⁶ SHEP-2 and SHEP-21N cells contained equivalent amounts of protein (data not shown). Protein synthesis rates were analyzed by ³⁵S-methionin incorporation. No differences were observed between SHEP-2 and N-myc expressing SHEP-21N cells. Also manipulation of the N-myc expression in SHEP-21N in a time course experiment did not reveal any difference in protein synthesis rates (data not shown). This suggests that the protein synthesis rate in SHEP-21N is either limited by a factor not induced by N-myc, or that protein synthesis is already maximal in the SHEP neuroblastoma cell line and beyond a level that can be boosted by N-myc.

### SAGE libraries of neuroblastomas with and without amplification of endogenous N-myc

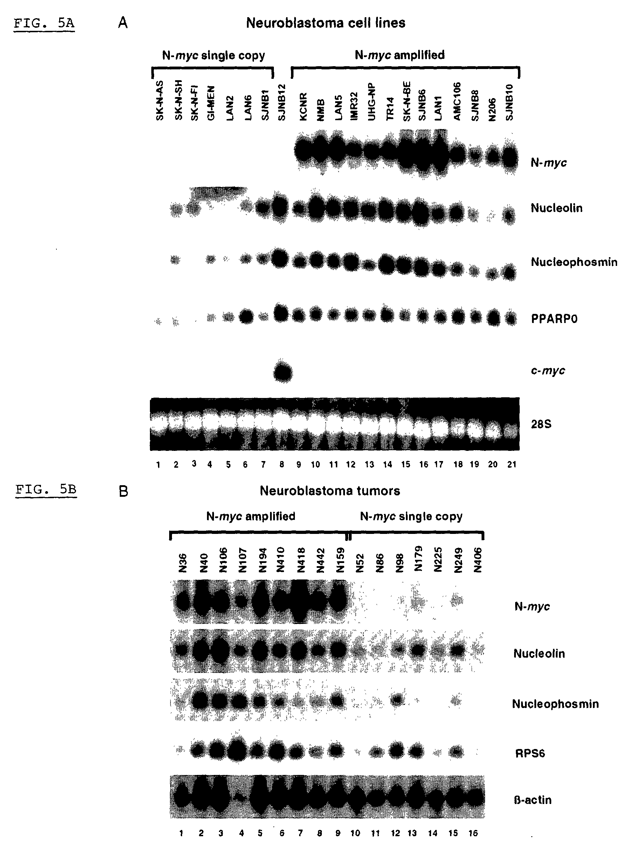

The SHEP neuroblastoma cell line has no endogenous N-*myc* expression, therefore the N-*myc* transfected cells do not necessarily have a genetic background representative for N-*myc* amplified neuroblastomas. For example, 90% of the N-*myc* amplified neuroblastomas have deletions of the chromosomal region 1p35-36 (Caron *et al*., 1993), while the SHEP-2 and SHEP-21N cells have two apparently intact p arms of chromosome 1 (data not shown). To address the question whether the here identified downstream pathway of N-myc is also *in vivo* activated, we generated SAGE libraries of two neuroblastomas. Neuroblastoma tumor N159 has N*-myc* amplification and expression and neuroblastoma N52 is an N-*myc* single copy tumor without N-*myc* expression (Fig. 5B, lanes 9 and 10). We sequenced 39.598 tags of the two libraries. The tag frequencies were normalized per 20,000 tags and compared. N-*myc* was represented by 16 tags in N159 and 0 tags in N52. There are 52 tags differentially expressed (p<0.01) in the libraries. These differences are probably only partly caused by N-myc, as the two tumors are likely to differ in more aspects. We analyzed which of the N-myc target genes identified in the SHEP cells did correlate with N-myc in the two tumors.

The 56 significantly (p<0.01) induced ribosomal protein genes detected in SHEP-21N produce a total of 988 tags in N52 and 1600 tags in N159 (per 20.000 tags). The N-*myc* amplified N159 tumor therefore has a 62% higher ribosomal protein gene expression. There are 36 tags with an increase of at least 50% and 22 tags with an increase of at least 100% in N159 compared to N52 (Figure 2B). These increases are more moderate than in the SHEP-21N cells (compare Fig. 2A and 2B), but strongly suggest that N-myc induces ribosomal protein gene expression *in vivo.* Also other genes functioning in protein synthesis are upregulated in N159. Increased expression in N159 compared to N52 is seen for nucleophosmin (from 4 to 19.2 tags), nucleolin (3 to 9 tags), eukaryotic translation initiation factor 4A, isoform 1 (4 to 9 tags) and the translation elongation factors EEF1a1 (50 to 96 tags) and EEF1g (18.4 to 32.8 tags). There is almost no induction of the genes involved in glycolysis and oxidative phosphorylation. The expression levels of five representative genes were confirmed by hybridization of Northern blots with total RNA from N159 and N52 (Fig. 5B and data not shown). These results show that the expression levels of many of the N-myc target genes identified in the SHEP-21N cells are also *in vivo* correlated with N-*myc* amplification and overexpression. However, this does not hold for all genes, suggesting that other factors modulate the activity of N-myc target genes.

### N-myc target gene expression analyzed in panels of neuroblastoma cell lines and tumors

To further analyze the induction of N-myc downstream genes in neuroblastoma, we examined their expression in a panel of neuroblastoma cell lines and tumors. Hybridization of a Northern blot of total RNA from 21 neuroblastoma cell lines showed a fair albeit imperfect correlation between expression of N*-myc,* nucleolin, nucleophosmin and the ribosomal protein PPARP0 (Figure 5A). Cell line SJNB12 has no N-*myc* expression, but a very high expression of the N-myc target genes. However, this cell line has c-*myc* amplification and over-expression (Figure 5A, lane 7 and Cheng *et al*., 1995), suggesting that c-myc may induce the same target genes as N-myc (see below).

As cell lines are not fully representative for neuroblastoma tumors *in vivo,* we analyzed 16 fresh neuroblastomas including the aggressive stages 3 and 4 and the less aggressive stages 1, 2 and 4s. A Northern blot analysis showed a fair overall correlation between expression of N*-myc,* nucleolin and nucleophosmin (Figure 5B). There are some exceptions, but the overall results suggest that nucleolin and nucleophosmin are also *in vivo* targets of N-myc induction. Ribosomal protein S6 (RPS6) expression showed a less consistent relationship with N-*myc,* indicating that besides N-myc also other factors may modulate its expression.

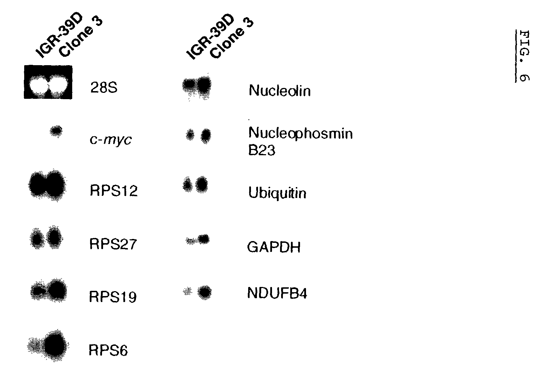

Several N-myc target genes are induced or suppressed by c*-myc* as well N*-myc* belongs to the same family of proto-oncogenes as c*-myc.* Since both oncogenes induce similar phenotypic effects and share several target genes, we analyzed whether the N-myc downstream targets identified in this study are targets of c*-myc* as well. We therefore analyzed the melanoma cell line IGR39D and a c-*myc* transfected clone of this cell line (clone 3, Versteeg *et al.,* 1988). Northern blots with total RNA of these cell lines were hybridized with the 26 probes tested on the SHEP-2 and SHEP-21N cells. Nine of 23 N-myc induced targets appeared to be induced by c*-myc* as well (Figure 6). They are the ribosomal protein genes S12, S27, S19, S6 and nucleolin, nucleophosmin, ubiquitin, GAPDH and NDUFB4. Three of the N-myc-suppressed targets were tested and found to be suppressed by c-myc as well. They were Osteonectin (No. 118/123/125), Plasminogen activator inhibitor type 1 (No. 112) and connective tissue growth factor (No 127). Therefore, c-myc and N-myc share about 46% of their target genes in the here tested cell systems. Interestingly, nucleophosmin, nucleolin and most ribosomal protein genes are among them.

We found induction of 86 transcripts contributing to ribosome biogenesis, mRNA translation, protein maturation and protein turnover, demonstrating that enhancement of protein synthesis is a major function of N-myc. We found a striking 45% higher rRNA content in SHEP-21N than in SHEP-2. There was no overall increase in the rate of protein synthesis in SHEP-21N. One interpretation is that some rate limiting components of the protein synthesis machinery are not induced in SHEP-21N cells. The SAGE libraries of the N-*myc* single copy neuroblastoma N52 and the N-*myc* amplified tumor N159 showed that ribosomal protein genes, nucleolin and nucleophosmin and five translation initiation and elongation factors are over-expressed in the N-*myc* amplified neuroblastoma *in vivo.* The Northern blot analysis of 37 neuroblastomas and neuroblastoma cell lines further confirmed induction of these genes in N-myc amplified neuroblastoma. These results show that myc genes function as major regulators of protein synthesis. This is in line with the reduced rate of protein synthesis in fibroblasts with a homozygous inactivation of c-*myc* (Mateyak et al., 1997) and the increased protein synthesis in fibroblast after activation of c-myc (Scmidt, 1999).

### Energy production, mitochondria and apoptosis

The two other comprehensive sets of N-myc downstream target genes are implicated in the glycolysis and the mitochondrial electron transfer and ATP synthesis pathway. The identification of the electron transfer and ATP synthesis pathway as a major target of N-myc induction bears on the relationship between the mitochondrial transmembrane potential and apoptosis. Mitochondria have two faces: they provide the energy for fast cycling cells and they can drive the cell into apoptosis. Similarly, the myc oncogenes can induce vigorous cell proliferation as well as massive apoptosis. N-myc expression renders SHEP-21N cells susceptible to apoptotic triggers (Fulda et al., 1999; Lutz et al, 1998). Many key events in apoptosis focus on mitochondrial membrane potential (reviewed in Green and Reed, 1998). Examples are cytochrome c release, hyperpolarization of the inner membrane, opening of the permeability transition pore and generation of reactive oxigen species (ROS). During normal electron transport in the mitochondrial membrane, 1 to 5% of the electrons lose their way and generate ROS. Any interruption of the electron transfer pathway strongly increases ROS production, with a deleterious effect on the cell (Kroemer et al., 1997). Enhancement of the electron flow by N-myc would upon interruption of the electron transfer chain boost ROS production. In addition, the moderate up-regulation of VDAC (Figure 1) could stimulate cytochrome c release and apoptosis. Therefore, N-myc induction of the electron transfer genes logically provides the energy required for cell proliferation. Meanwhile, it could increase the deadly potential of the mitochondria and upon triggering tip the scale towards execution of apoptosis.

Interestingly, tags for oxidative phosphorylation pathways are not over-expressed in the N*-myc* amplified N159 tumor. This tumor might have been selected *in vivo* for additional defects, that interfere with part of the N-myc downstream pathway. While SHEP-21N cells expressing N-myc are susceptible to apoptotic triggers (Lutz et al., 1998), neuroblastoma cell lines with overexpression of endogenous N-*myc* are refractory to such triggers. This shows that these cell lines have defects in the pro-apoptotic arm of the N-myc downstream pathway. It will be interesting to analyze whether this relates to the lack of induction of mitochondrial protein genes.

### N-myc and c-myc share target genes

To date, only two target genes of N-myc have been published, which are targets of c-myc as welL (Lutz et al., 1996; Eilers et al., 1991; Bello-Fernadez et al., 1993). Of the 23 upregulated targets of N-myc that we tested on Northern blots, 9 are induced by c-*myc* in transfected melanoma cells. Both down-regulated N-myc targets that we tested were also downregulated by c-myc. Since the N-myc induced downstream pathway genes form very concise functional groups of genes, we postulated that N-myc functions as a general stimulator of protein synthesis and energy production. Since c-myc has an equally powerful growth-inducing and transforming effect as N-myc, it is difficult to envisage that c-myc would only induce a subset of the genes that are necessary to boost the protein and ATP synthesis machinery's. It appears more likely that N-myc and c-myc activate the same basic cellular functions. Indeed, c-myc is implicated in induction of protein synthesis in fibroblasts cell lines (see above). We observed that induction of genes by N-myc strongly depends on their basic expression levels (fig.2). It is therefore possible that high expression of potential target genes in the original melanoma cell line may have prevented their induction by c-myc.

The physiological role of myc genes has been enigmatic, as only very few target genes were identified thus far. Here we describe 284 transcripts that are target of N-myc, some of which are targets of c-myc induction as well. These results show that myc genes function as major regulators of protein synthesis and cellular energy production. It is likely that this induction mediates the enhanced transition through the G1 phase of the cell cycle in normally proliferating cells and in cells that are induced to proliferate by physiological stimuli. The effect on protein synthesis confirms earlier postulations based on the identification of a limited set of target genes (Schmidt, 1999, Mateyak et al., 1997, Johnston et al., 1999). The stimulatory effect on genes in the electron transfer and ATP synthesis pathway is unexpected and fits well with the energy requirements for enhanced protein production and G1 transition and could relate to the well established apoptotic effect of *myc* genes.

List of tags and genes induced or suppressed by N-myc

Table 1 lists all tags that we found to be significantly (p<0.01) induced or suppressed by N-myc in the comparison of the SHEP-2 and SHEP-21N SAGE libraries. The comparison is base on 21,559 tags of SHEP-21N and 44,674 tags of SHEP-2. The tag frequencies shown are normalized per 10.000 tags (column SHEP-2 and SHEP-21N). The column "ratio ON:OFF" shows the fold induction (positive values) or suppression (negative values) by N-myc. When a tag had a zero expression in one of the libraries, we assumed for ratio calculation that the tag was present one time in the entire library. The Unigene numbers of the National Center for Biotechnology Information (NCBI, Bethesda, USA) are given in the column "Unigene". The numbers are based on the NCBI Unigene database as by 29-3-2000. The next column shows the Unigene description. Furthermore, for each Unigene cluster, one or two Genbank accession codes are given. For some tags, we identified two possible corresponding genes. This is indicated by an asterix in the column next to the tag.

### Experimental Procedures

### Cell lines

Neuroblastoma cell lines and culture conditions were as described before (Cheng *et al.*, 1995). The melanome cell lines IGR39D and clone 3 were described by Versteeg *et al.* (1988). The SHEP cell lines were maintained in RPMI 1640 medium supplemented with 10% fetal calf serum, 4 mM L-glutamine, 100U/ml penicillin and 100 µg/ml streptomycin (Lutz *et al.,* 1996). Tetracycline (Sigma) was used at a concentration of 10 ng/ml medium to inhibit N-*myc* expression.

### Generation of SAGE libraries

SAGE was performed as described by Velculescu *et al.* (1995) with a few adaptations. Total RNA was extracted by guanidium thiocyanate (Chromczynski and Sacchi, 1987). Poly(A)⁺ RNA was isolated using the MessageMaker kit (Gibco/BRL) according to the manufacturer's instructions. SAGE libraries were generated using minimally 4 µg poly(A)⁺ RNA. The cDNA was synthesized according to the Superscript Choice System (Gibco/BRL), digested with *Nla*III and bound to streptavidine-coated magnetic beads (Dynal). Linkers containing recognition sites for *Bsm*FI were ligated to the cDNA. Linker tags including a cDNA tag were released by *Bsm*FI digestion, ligated to one another and amplified. The PCR products were heated for 5 min at 65°C before preparative analysis on a polyacrylamide gel. After the ligation into concatameres a second heating step was included (15 min at 65°C) and fragments between 800bp and 1500bp were purified and cloned in pZero-1 (Invitrogen). Colonies were screened with PCR using M13 forward and reverse primers. Inserts larger than 300bp were sequenced with a BigDye terminator kit and analyzed on a 377 ABI automated sequencer (Perkin Elmer).

### Analysis SAGE database

The SAGE libraries were analyzed using the SAGE 300 program software package (Velsculescu *et al.,* 1997). P-values were calculated using Monte Carlo simulations.Transcripts were identified by comparison of the tags in the database with the "tag to gene map" (SAGEmap) from Cancer Genome Anatomy Project at the NCBI (http://www.ncbi.nlm.nih.gov/SAGE). This database links Unigene clusters to SAGE tags (Lal *et al.,* 1999). The gene assignments were subsequently checked by hand for sequencing errors causing incorrect tags and for erroneous gene assignments based on hybrid Unigene clusters. Other database analyses and generation of specific primers utilized the Wisconsin GCG package software.

### Northern Blot analysis

Total RNA (20 µg per lane) was electrophoresed through a 0.8% agarose gel in the presence of 6.7 % formaldehyde and blotted on Hybond N membranes (Amersham) in 10 x SSC. Hybridization was carried out overnight in 0.5 M NaHPO₄, pH 7.0, 7 % SDS, 1 mM EDTA at 65°C. Filters were washed in 40 mM NaHPO₄, 1% SDS at 65°C. Probes were labelled by random priming of sequence-verified PCR products. A complete list of all the primers used in RT-PCR reactions is available on request.

### Total Protein content

Exponentially growing cells were harvested and cell number was determined using a Coulter counter. Cells (1 x 10⁶) were lysed in 20 mM Tris-HCl (pH 8.0), 137 mM NaCl, 10% glycerol, 1% NP40 and protease inhibitors (protease cocktail, Roche). Samples were assayed with the Bio-Rad Protein assay. Assays were performed at least in duplicate.

### Western Blots

Cell lysates were separated on SDS-polyacrylamide gel and electroblotted onto Immobilon-P transfer membrane (Millipore). Blocking of the membrane and incubation with antibodies involved standard procedures. Proteins were visualized using the ECL detection system (Amersham). Anti-nucleophosmin monoclonal antibody was a gift of dr. P.K. Chan (Baylor College of Medicine). The antibody against nucleolin was a gift of Dr. P. Bouvet (CNRS, IPBS, Toulouse, France). Anti-N-myc was obtained from PharmIngen (Clone B8.4.B).

### Total rRNA content

Total RNA of 1 x 10⁶ exponentially growing cells was extracted by guanidium isothiocyanate (Chromczynski and Sacchi, 1987) and photospectrometrically quantified. Results of ten isolations of each of the cell lines SHEP-2 and SHEP-21N were statistically analyzed with the Students T test for independent samples. Aliquots on a per cell basis were subjected to agarose gel electrophoresis and stained with ethidium bromide. The relative fluorescence of the rRNA bands was quantified using the Kodak Digital Science 1D Image Analysis Software package (EDAS 120).

### References

Alex, R, Sozeri O, Meyer S, Dildrop R. (1992). Determination of the DNA sequence recognized by the bHLH-zip domain of the N-Myc protein. Nucleic Acids Res. 20, 2257-2263.
Amati, B., Alevizopolous, K., Vlach. (1998). Myc and the cell cycle. Front. Biosci 3, D250-D268.
Bello-Fernandez, C., Packham, G., Cleveland, J.L. (1993). The ornithine decarboxylase gene is a transcriptional target of c-Myc.Proc. Natl. Acad. Sci. USA 90, 7804-7808.
Benvenisty, N., Leder, A., Kuo, A., Leder, P. (1992). An embryonically expressed gene is a target for c-Myc regulation via the c-Myc-binding sequence. Genes Dev. 6, 2513-2523.
Bernards, R., Dessain, S.K., Weinberg, R.A. (1986). N-myc amplification causes down-modulation of MHC class I antigen expression in neuroblastoma. Cell 47, 667-674.
Blackwood, E.M., Kretzner, L., Eisenman, R.N. (1992). Myc and Max function as nucleoprotein complex. Curr. Opn. Genet. Dev. 2, 227-235.
Borer, R.A., Lehner, C.F., Eppenberger, H.M., Nigg, E.A. (1989). Major nucleolar proteins shuttle between nucleus and cytoplasma. Cell 56, 379-390.
Bouvet, P., Diaz, J.J., Kindbeiter, K., Madjar, J.J., Amalric., F. (1998). Nucleolin interacts with several ribosomal proteins through its RGG domain. Journal of Biol. Chem. 278, 19025-19029.
Boyd, K.E., Farnham, P.J. (1997). Myc versus USF: discrimination at the cad gene is determined by core protein elements. Mol. Cell. Biol. 17, 2529-2537.
Caron, H., van Sluis, P., van Hoeve, M., de Kraker, J., Bras, J., Slater, R., Mannes, M., Voute, P.A., Westerveld, A., Versteeg, R. (1993). Allelic loss of chromosome 1p36 in neuroblastoma is of preferential maternal origin and correlates with N-myc amplification. Nature Genetics 4, 187-190.
Chan, PK, Aldrich, MB, Yung, BYM (1987). Nucleolar protein B23 translocation after doxorubicin treatment in murine tumor cells. Cancer Res. 47, 3798-3801.
Chan, PK, Aldrich, M, Chakrabarty, S, (1988). Assessment of tumor cell sensitivity to mitomycin C by B23 translocation assay. Cancer Lett. 40, 143-49.
Chan, PK, Characterization and cellular localization of nucleophosmin/B23 in HELA cells treated with cytostatic agents. Exp. Cell Res. 203, 174-181.
Chan, P.K., Chan, F.Y., Morris, S.W., Xie Z. (1997). Isolation and characterization of the human nucleophosmin/B23 (NPM) gene: identification of the YYlbinding site at the 5' enhancer region. Nucleic Acids Res 25, 1225-1232.
Cheng, N.C., Van Roy, N., Chan, A., Beitsma, M., Westerveld, A., Speleman, F., Versteeg, R. (1995). Deletion mapping in neuroblastoma cell lines show two distinct tumor suppressor genes in the 1p35-36 region, only one of which is associated with N-myc amplification. *Oncogene 10, 291-297.*
Chomczynski, P., Sacchi, N. (1987). Single-step method of RNA isolation by acid guanidiniumthiocyanate-phenol-chloroform extraction. Anal Biochem 162, 156-159.
Chung, S., Perry, R.P. (1993). The importance of downstream delta-factor binding elements for the activity of the rpL32 promoter. Nucleic Acids Research 21, 3301-3308.
Cohen, MB, Glazer, RI (1985). Comparison of the cellular and RNA-dependent effects of sangivamycin and toyocamycin in human colon carcinoma cells. Mol Pharmacol 27, 349-55.
Cole, MD, (1986). The myc oncogene: its role in transformation and differentiation. Annu Rev Genet. 20, 361-84.
Cole , M.D., McMahon, S.B. (1999). The Myc oncoprotein: a critical evaluation of transactivation and target gene regulation. Oncogene 18, 2916-2924.
Cooper, H.L., Gibson, E.M. (1971). Control of synthesis and wastage of ribosomal ribonucleic acid in lymphocytes. II. The role of protein synthesis.J. Biol. Chem. 246, 5059-5066.
Daskis, J.I., Lu, R.Y., Facchini, L.M., Marhin, W.W., Penn, L.J.Z. Myc induces cyclin D1 expression in the adsence of the novo protein synthesis and links mitogen-stimulated signal transduction to the cell cycle. (1994). Oncogene 9, 3635-3645.
Dang, CV and Lee, LA (1995). C-Myc function in neoplasia. R.G. Landes and Springer Verlag, Austin Texas.
Dang, C.V. (1999). C-Myc target genes involved in cell growth, apoptosis and metabolism. Mol. Cell. Biol. 19, 1-11.
Dudov, K.P., Dabeva MD. (1983). Post-transcriptional regulation of ribosome formation in the nucleus of regenerating rat liver. Biochem J. 210, 183-192.
Eilers, M., Schrim, S., Bishop, J.M. (1991). The myc protein activates transcription of the alpha-prothomyosin gene. EMBO Journal 10, 133-141.
Evan, G.I., Wyllie, A.H., Gilbert, C.S., Littlewood, T.H., Land, H., Brooks, M., Waters, C.M., Penn, L.Z., Hancock, D.C. (1992). Induction of apoptosis in fibroblasts by c-myc protein. Cell 69,119-128.
Fulda, S., Lutz, W., Schwab, M., Debatin, K.M. (1999). MycN sensitizes neuroblastoma cells for drug-induced apoptosis. Oncogene 18, 1479-1486.
Galaktionov, K., Chen, X., Beach, D. (1996). Cdc25 cell cycle phosphatase as a target of c-myc. Nature 382, 511-517.
Gamberi, G, Benassi, MS, Bohling, T, Ragazzini, P, Molendini, L, Solazzo, MR, Merli, M, Ferrari, C, Magagnoli, G, Bertoni, F, Picci, P. (1998). Prognostic relevance of c-myc gene expression in giant-cell tumor of bone. J. of Orthopaedic Res. 16, 1-7.
Ginisty, H., Almalric, F., Bouvet, P. (1998). Nucleolin functions in the first step of ribosomal RNA processing. EMBO Journal 17, 1476-1486.
Ginisty, H., Sicard, H., Roger, B., Bouvet, P. (1999). Structure and function of nucleolin. J. Cell Sci. 112, 761-772.
Grandori, C., Mac, J., Siebelt, F., Ayer, D.E., Eisenman, R.N. (1996). Muyc-Max heterodimers activate a DEAD box gene and interact with multiple E box-related sites in vivo. EMBO Journal 15, 4344-4357.
Greasley, P.J., Bonnard, C., Amati, B. (2000) Myc induces the nucleolin and BN51 genes: possible implications in ribosome biogenesis. Nucleic Acid Research 28, 446-453.
Green, D., Reed, J.C. (1998). Mitochondria and apoptosis. Science 281,1309-1312.
Hanson, K.D., Schichiri, M., Follansbee, M.R., Sedivy, J.M. (1994). Effects of c-myc expression on cell cycle progression. Mol. Cell. Biol. 14, 5748-5755.
Hariharan, N., Kelley, D.E., Perry, R.P. (1991). Delta, a transcription factor that binds to downstream elements in several polymerase II promoters, is a functionally versatile zinc finger protein. Proc. Natl. Acad. Sciences 88, 9799-9803.
Henriksson, M., and Luscher, B. (1996). Proteins of the Myc network: essential regulators of cell growth and differentiation. Adv. Ancer Res. 68, 109-182
Jansen-Durr, P., Meiche, A., Steiner, P., Pagano, M., Finke, K., Botz, J., Wessbecher, J., Draetta, G., Eilers, M. (1993). Differential modulation of cyclin gene expression by Myc. Proc. Natl. Acad. Sci. USA 90, 3685-3689.
Johnston, L.A., Prober, D.A., Edgar, B.A., Eiseman, R.N., Gallant, P. (1999). Drosophila myc regulates cellular growth during deveopment. Cell 98, 779-790.
Jones, R.M., Branda, J., Johnston, M., Polymensis, Gadd, M., Rustgi, A., Callanan, L., Schmidt, E.V. (1996). An essential E box in the promoter of the gene encoding the mRNA cap-binding protein (eukaryotic initiation factor 4E) is a target for activation by c-myc. Mol. Cell. Biol. 16, 4754-4764.
Kakisako, K, Miyahara, M, Uchino S, Adachi, Y, Kitano S, (1998). Prognostic significance of c-myc mRNA expression assessed by semi-quantitative RT-PCR in patients with colorectal cancer. Oncology Reports 5, 441-5.
Komarov, PG, Komarova, EA, Kondratov, RV, Christov-Tselkov, K, Coon, JS, Chernov, MV and Gudkov, AV (1999). Science 285, 1733-37.
Kroemer, G., Zamzami, N and Susin, SA (1997). Mitochondrial control of apoptosis. Immun. Today 18, 45-51.
Lane, T.F. and Sage, E.H. (1994). The biology of SPARC, a protein that modulates cell-matrix interactions. FASEB J. 8, 163-173.
Lal A., Lash, A.E., Altschul, S.F., Velculescu, V., Zhang, L., McLendon, R.E., Marra, M.A., Prange, C., Morin, P.J., Polyak, K., Papadopoulos, N., Vogelstein, B., Kinzler, K.W., Strausberg, R.L., Riggins, G.J. (1999). A public database for gene expression in human cancers. Cancer Res 59, 5403-7.
Lutz, W., Stöhr, M., Schürmann, J., Wenzel, A., Löhr, A., Schwab, M. (1996). Conditional expression of N-*myc* in human neuroblastoma cells increases expression of α-prothymosin and ornithine decarboxylase and accelerates progression into S-phase early after mitogenic stimulation of quiescent cells. Oncogene 13, 803-812.
Lutz, W., Fulda, S., Jeremias, I., Debatin, K.M., Schwab, M. (1998). MycN and IFNgamma cooperate in apoptosis of human neuroblastoma cells. Oncogene 17, 339-346.
Ma, A., Moroy T., Collum R., Weintraub H., Alt F.W., Blackwell, T.K. (1993). DNA binding by N- and L-Myc proteins. Oncogene 8, 1093-1098.
Marcu KB. Bossone SA. Patel AJ.(1992). myc function and regulation. Annu Rev Biochem. 61, 809-60.
Marx N. Bourcier T. Sukhova GK. Libby P. Plutzky J. (1999). PPARgamma activation in human endothelial cells increases plasminogen activator inhibitor type-1 expression: PPARgamma as a potential mediator in vascular disease. Arterioscler Thromb Vasc Biol. 19, 546-51.
Mateyak, M.K., Obaya, A.J., Adachi, S., Sedivy, J.M. (1997). Phenotypes of c-Myc-deficient rat fibroblasts isolated by targeted homologous recombination. Cell. Growth Differ. 8, 1039-1048.
Morris, S.W., Kirstein, M.N., Valentine, M.B., Dittmer, K.G., Shapiro, D.N., Saltman, D.L., Look, A.T. (1994). Fusion of a kinase gene, ALK, to a nucleolar protein gene, NPM, in non-Hodginkin's lymphoma. Science 263, 1281-1284.
Nicoloso, M., caizergues-Ferrer, M., Michot, B., Azum, M.C., Bachellerie, J.P. (1994). U20, a novel small nucleolar RNA, is encoded in an intron of the nucleolin gene in mammals. Mol Cell Biol 14, 5766-5776.
Nicoloso, M., Qu, L.H., Michot, B., Bachellerie, J.P. (1996). Intron-encoded, antisense small nucleolar RNAs: the characterization of nine novel species points to their direct role as guides for the 2'-O-ribose methylation of rRNAs.
Nisen PD. Zimmerman KA. Cotter SV. Gilbert F. Alt FW. (1986). Enhanced expression of the N-myc gene in Wilms' tumors. Cancer Res. 46, 6217-22.
Olson, M.O. (1991). The role of protein in nucleolar structure and function in *The Eukaryotic Nucleus:* Molecular Biochemistry and Macromolecular Assemblies (Strauss, P.R., Wilson, S.H. eds.) Vol. 2, 541-546. Telford Press, Caldwell, N.J.
Pandolfi, P.P. (1996). PML, PLZF and NPM genes in the molecular pathogenesis of acute promyelocytic leukemia. Haematologica 5, 472-482.
Perlaky, L, Valdez, BC, Busch, H (1997). Effects of cytotoxic drugs on translocation of nucleolar RNA helicase RH-II/Gu. Exp. Cell Research 235, 413-20.
Peter, M., Nakagawa, J., Doree, M., Labbe, J.C., Nigg, E.A. (1990). Identification of major nucleolar proteins as candidate mitotic substrates of cdc2 kinase. Cell 60, 791-801.
Philipp, A., Schneider, A., Varsik, I., Finke, K., Xiong, Y., Beach, D., Alito, K., Eilers, M. (1994). Repression of cyclin D1: a novel function of Myc. Mol. Cell. Biol. 14, 4032-4043.
Polyak, K., Xia, Y, Zweier, JL, Kinzler, K and Vogelstein, B (1997) A model for p53-induced apoptosis. Nature 389, 300-305.
Qu, L.H., Nicoloso, M., Michot, B., Azum, M.C., Caizergues-Ferrer, M., renalier, m.h., Bachellerie, J.P. (1994). U21, a novel small nucleolar RNA with a 13nt. Complementarity to 28S rRNA, is encoded in an intron of ribosomal protein L5 gene in chicken and mammals. Nucleic Acid Research 22, 4073-4081.
Redner, R.L., Rush, E.A., Faas, S., Rudert, W.A., Corey, S.J. (1996). The t(5;17) variant of acute promyelocytic leukemia expresses a nucleophosmin-retinoic acid receptor fusion. Blood 87, 882-886.
Rosenwald, I. B., Rhoads, D.B., Callanan, L.D., Isselbacher, K.J., Schmidt, E.V. (1993). Increased expression of eukaryotic translation initiation factors eIF-4E and eIF-2 alpha in response to growth induction by c-myc. Pro. Natl. Acad. Sci. USA 90, 6175-6178.
Savkur, R.S., Olson, M.O.J. (1998). Preferential cleavage in pre-ribosomal RNA by protein B23 endonuclease. Nucleic Acid Research 26,4508-4515.
Seeger, R.C., Brodeur, G.M., Sather, H., Dalton, A., Siegel, S.E., Wong, K.Y., Hammond, D. (1985). Association of multiple copies of the N-myc oncogene with rapid progression of neuroblastomas. N. Engl. J. Med. 318, 1111-1116.
Schwab, M., Alitalo, K., Klempnauer, K.H., Varmus, H.E., Bishop, J.M., Glibert, F., Brodeur, G.M., Golsdstein, M., Trent, J.M. (1983). Amplified DNA with limited homology to myc cellular oncogene is shared by human nueroblastoma cell lines and a neuroblastoma tumour. Nature 305, 245-248.
Shimizu, S., Narita, M., Tsujimoto, Y. (1999). Bcl-2 family proteins regulate the release of apoptogenic cytochrome c by the mitochondrial channel VDAC. Nature 399, 483-487.
Schlagbauer-Wadl, H, Griffioen, M, van Elsas, A, Schrier, PI, Pustelnik, T, Eichler, HG, Wolff, K, Pehamberger, H, Jansen, B (1999). Influence of increased c-myc expression on the growth characteristics of human melanoma. J Invest Dermatol 112, 332-6.
Schmidt, E.V. (1999). The role of c-myc in cellular growth. Oncogene 18, 2988-2996).
Solomon, D.L.C., Philipp, A., Land, H., Eilers, M. (1995). Expression of cyclin D1 mRNA is not upregulated by Myc in Rat fibroblasts. Oncogene 11, 1893-1897.
Sierra, A, Castelsague, X, Escobedo, A, Moreno, A, Drudis, T, Fabra, A. (1999). Synergistic cooperation between c-myc and Bcl-2 in lymph node progression of T1 human breast carcinomas. Breast Cancer Res. Treat. 54, 39-45.
Steiner, P., Philipp, A., Lukas, J., Godden-Kent, D., Pagano, M., Mittnacht, S., Bartek, J., Eilers, M. (1995). Identification of a Myc-dependent step during the formation of active G1 cyclin-cdk complexes. EMBO Journal 14, 4814-4826.
Strojan, P, Budihna, M, Smid, L, Skrk, J (1998). Urokinase-type plasminogen activator (uPA) and plasminogen activator inhibitor type 1 (PAI-1) in tissue and serum of head and neck squamous cell carcinoma patients. Eur J Cancer 34, 1193-7.
Szebeni, A., Olson, M.O. (1999). Nucleolar protein B23 has molecular chaperone activities. Protein Sci 8, 905-912.
Tujeta, R., Tujeta, N. (1998). Nucleolin: a multifunctional major nucleolar phosphoprotein. Crit. Rev Biochem Mol Biol 33, 407-436.
Velculescu, V.E., Zhang, L., Vogelstein, B., and Kinzler, K.W. (1995). Serial analysis of gene expression. Science 270, 484-487.
Velculescu, V.E., Zhang, L., Zhou, W., Volgelstein, J., Basrai, M.A., Basset Jr., D.E., Hieter, P., Volgelstein, B., and Kinzler, K.W. (1997). Characterization of the yeast transcriptome. Cell 88, 243-251.
Versteeg, R., Noordermeer I.A., Krüse-Wolters, M., Ruiter, D.J., and Schrier, P.I. (1988). C-myc down-regulates class I HLA expression in human melanomas. *EMBO J. 7, 1023-1029.*
Weiss, W.A., Aldape, K., Mohapatra, G., Feuerstein, B.G., Bishop, J.M. (1997). Targeted expression of MYCN causes neuroblastoma in transgenic mice. EMBO Journal 16, 2985-2995.
Welle, S., Bhatt, K., Thornton, C.A. (1999). Inventory of High-Abundance mRNAs in Skeletal Muscle of Normlal Men. Genome Research 9, 506-513.
Wiedmann, B., Sakai, H., Davis, T.A., Wiedmann, M. (1994). A proteincomplex required for signal-sequence-specific sorting and translocation. Nature 370, 434-440.
Wilson, W. (1968). Phase I study with toyocamycin (NSC-63701). Cancer Chemotherapy Reports 52, 301-3.
Wool, I., Chan, Y.L., Glück, A., (1996). In Translational Control, (Hershey, J., Mathews, M., and Sonenberg, N., eds), 685-732, Cold Spring Harbor Laboratory Press, Cold Spring Harbor, NY.
Yung, BYM, Busch, H, Chan, PK (1985). Translocation of nucleolar phosphoprotein B23 (37 kDa/pI 5.1) induced by selective inhibitors of ribosome synthesis. Biochim. Biophys. Acta. 826, 167-173.
Yung, BYM, Bor, AMS, Chan, PK (1990). Short exposure to actinomycin D induces reversible translocation of protein B23 as well as reversible inhibition of cell growth and RNA synthesis in HeLa cells. Cancer Res. 50, 5987-91.

### Legends to the figures:

Figure 1. Northern blot analysis of N-myc downstream target genes. Equal amounts of total RNA from exponentially growing SHEP-2 and SHEP-21N cells were loaded. Northern blots were hybridized with probes for the 23 indicated N-myc targets.
Figure 2. Level of induction of the 56 ribosomal protein genes identified as N-myc targets (p<0.01) in SHEP-21N cells. A. Fold induction by N-myc in SHEP-21N as a function of the basic expression levels in SHEP-2. X-coordinate: basic expression level in SHEP-2 normalized per 10.000 tags. Y-coordinate: fold induction in SHEP-21N. B. Increase of the same 56 ribosomal protein genes in N*-myc* amplified neuroblastoma N159 as a function of the basic expression level in N-*myc* single copy neuroblastoma N52. X coordinate: expression level in N52 normalized per 10.000 tags. Y coordinate: Fold increase in N159 relative to N52.
Figure 3. Time-course analysis of N*-myc* and downstream target gene induction in SHEP-21N cells. SHEP-21N cells were treated for 24 hours with 10ng/ml tetracycline, washed and grown for an additional 36 hours without tetracycline. Cells were harvested at 0 hr, 8hr and 24 hr of tetracyclin treatment. Subsequent samples were taken at 1 hr, 2 hr, 4 hr, 8 hr, 10 hr, 12 hr, 24 hr and 36 hr after removal of the antibiotic. A: Northern blot analysis of total RNA at indicated time points. B: Western blot analysis of N-myc protein at indicated time points. Ten mg of total protein samples of the time-course experiment were fractionated through a 10% SDS-PAGE gel, blotted on Immobilon membrane and probed with a monoclonal anti-N-myc antibody.
Figure 4. Nucleolin and Nucleophosmin protein expression and total RNA content of SHEP-2 and SHEP-21N. A: Western blot analysis of nucleolin, N-MYC and nucleophosmin protein expression. Total cell extracts (10 µg) were fractionated through an acrylamide gel, blotted and probed with polyclonal antibodies against nucleolin (upper panel) and monoclonal antibodies against N-myc (middle panel) and nucleophosmin (lower panel). Control cell lines IMR32 and SK-N-FI have high, respectively low expression of N-*myc,* nucleolin and nucleophosmin. B: Total RNA content of SHEP-2 and SHEP-21N. RNA was isolated from ten samples of 10⁶ cells of each cell line and photospectromerically analyzed. Error bars give the S.D.
Figure 5. Northern blot analysis of total RNA from neuroblastoma cell lines and tumors. Filters were hybridized with indicated probes. RNA quantification was checked by ethidium bromide staining, the 28S band is shown. A: panel of 21 neuroblastoma cell lines. B: Panel of 16 fresh tumors. Tumors in lanes 1-9 are N-myc amplified.
Figure 6. Northern blot analysis of induction of N-myc target genes in a c-myc-transfected melanoma cell line. Clone 3 is a c-myc transfected clone of the IGR39D melanoma cell line. Equal amounts of total RNA of IGR39D and clone 3 were loaded. Filters were hybridized with the indicated probes.

## Claims

1. A nucleic acid library comprising *myc*-dependent downstream genes or functional fragments thereof said genes essentially capable of supporting a neoplastic character of cancer such as growth, invasion or spread.

2. A library according to claim 1 wherein said *myc*-dependent downstream genes each comprises a nucleic acid essentially equivalent to a Tag sequence as shown in Table 1.

3. A library according to claim 1 or 2 wherein said myc-dependent downstream genes encode a ribosomal protein.

4. A library according to claim 1 or 2 wherein said myc-dependent downstream genes encode a protein related to protein synthesis.

5. A library according to claim 1 or 2 wherein said myc-dependent downstream genes encode a protein related to metastasis.

6. A library according to claim 1 or 2 wherein said myc-dependent downstream genes encode a glycolysis enzyme.

7. A library according to claim 1 or 2 wherein said myc-dependent downstream genes encode a mitochondrial functional protein.

8. A method for the treatment of cancer comprising modulating a *myc*-dependent downstream gene capable of supporting an essentially neoplastic character of said cancer.

9. A method according to claim 8 wherein said *myc*-dependent downstream gene comprises a nucleic acid functionally equivalent to a Tag sequence as shown in Table 1.

10. A method according to claim 8 or 9 wherein said cancer comprises *myc*-expressing tumour cells.

11. A method according to anyone of claims 8 to 10 wherein *myc* is N*-myc.*

12. A method according to claim 11 wherein said cancer comprises neuroblastoma.

13. A method for the diagnosis of cancer comprising detecting the relative presence or absence of a *myc*-dependent downstream gene capable of supporting an essentially neoplastic character of said cancer or gene product derived thereof.
